Europäisches Patentamt

European Patent Office

Office européen des brevets

(11) Publication number: **0 048 264**

**B1**

# EUROPEAN PATENT SPECIFICATION

(45) Date of publication of patent specification: **19.06.85**

(21) Application number: **81900905.1**

(22) Date of filing: **13.03.81**

(86) International application number:
**PCT/US81/00323**

(87) International publication number:
**WO 81/02670 01.10.81 Gazette 81/23**

(51) Int. Cl.⁴: **A 61 K 7/16,** A 61 K 7/18,
C 09 K 3/14

(54) DENTAL PROPHYLAXIS COMPOSITIONS AND THEIR USE.

(30) Priority: **17.03.80 US 131266**

(43) Date of publication of application:
**31.03.82 Bulletin 82/13**

(45) Publication of the grant of the patent:
**19.06.85 Bulletin 85/25**

(84) Designated Contracting States:
**DE FR GB**

(56) References cited:
DE-B-1 292 468
US-A-1 049 005
US-A-1 087 705
US-A-1 943 856
US-A-2 059 396
US-A-2 216 821
US-A-2 384 563
US-A-2 625 514
US-A-2 943 982 ·
US-A-3 105 013
US-A-3 450 813
US-A-3 855 147

(73) Proprietor: **PENNWALT CORPORATION**
**Pennwalt Building Three Parkway**
**Philadelphia Pennsylvania 19102 (US)**

(72) Inventor: **MUHLER, Joseph C.**
**P.O. Box 36**
**Howe, IN 46746 (US)**
Inventor: **PUT, Mark S.**
**4034 Westland Road**
**Fort Wayne, IN 46802 (US)**

(74) Representative: **Lambert, Hugh Richmond et al**
**D. YOUNG & CO. 10 Staple Inn**
**London, WC1V 7RD (GB)** ·

(56) References cited:
US-A-3 966 432
US-A-4 038 380
US-A-4 064 231
US-A-4 108 979
US-A-4 108 981
US-A-4 122 163
US-A-4 169 796
US-A-4 181 633

Courier Press, Leamington Spa, England.

# 0 048 264

## Description

### Field of the invention

The present invention relates to the field of dental prophylaxis and more specifically to prophylactic paste cleaning and polishing compositions.

These compositions have the ability to impart a smooth, highly polished surface to tooth enamel and to effectively remove all types of exogenous stains and accumulations from the teeth without resulting in undue abrasion of the enamel, dentin, or cementum. Reaccumulations of dental plaque and pellicle and occurrence and reformation of dental calculus on oral hard tissue are markedly reduced, thereby, significantly reducing the incidence of gingivitis and periodontal disease. Additionally, as a means of contributing to the partial control of dental caries, effective fluoride anticariogenic adjuvants, such as sodium fluoride, stannous fluoride, sodium monofluorophosphate, or acidulated phosphate fluoride, may be incorporated in such compositions. Other anticariogenic agents, such as aluminum carboxylates (U.S. Patent Nos. 4,042,680 and 4,108,981) may also be advantageously employed in such compositions.

### Description of the prior art

In the past, prophylactic pastes have been used for the removal of exogeneous stains that could not be removed by the routine use of a dentifrice and toothbrush. Even at present, many widely used dental prophylactic products contain pumice, silica, or other hard materials of a relatively large particle size in order to achieve fast and thorough cleaning. It is apparent that excessive abrasion and scratching of the enamel increase the rate of reformation of exogeneous stains and produce a low degree of enamel polish. It has been shown in several studies that a smooth, highly polished tooth surface is less receptive to reformation and retention of plaque, exogenous debris, pellicle, stains, and dental calculus.

Although significant improvements in the polishing efficacy of prophylactic compositions have been achieved in recent years through the use of zirconium silicate (U.S. Patents Nos. 3,330,732 and 3,378,445), alumina (U.S. Patent No. 3,670,076), and feldspar (U.S. Patent No. 3,892,843), these agents still fall short of imparting maximum levels of polish during a typical, short prophylactic treatment. Additionally, in order to achieve good cleaning properties with these materials, large particles are necessary; however, such particle sizes cause undesirably high abrasiveness and do not polish well. To obtain polishing, especially with alumina or zirconium silicate, it is necessary to include small-sized particles, which have little cleaning effect.

The beneficial effects, in terms of a reduction in the incidence of dental caries, resulting from the incorporation of water-soluble fluoride salts, such as sodium fluoride, stannous fluoride, sodium monofluorophosphate, or acidulated phosphate fluoride, are well known. However, effects to utilize such salts in prophylactic paste compositions have been handicapped by the tendency of fluoride and/or tin(II) ions to be inactivated and rendered unavailable by other ingredients, particularly the abrasive components of such compositions. In general, while prophylactic abrasives in therapeutic products used today are to varying degrees compatible with fluoride agents, there is a wide variation in compatibility. Abrasives containing polyvalent cations such as calcium and iron, either in their crystal structure or as impurities, are usually not particularly compatible.

In US 4,122,163 dentifrice compositions are disclosed comprising particles of calcined kaolin as the abrasive whilst in US 3,105,013 dentifrice compositions are disclosed comprising calcined aluminium silicate, both of which may be used in combination with fluoride. Whilst such abrasives are effective in dentifrices, particularly as polishing agents, they are ineffective in prophylactic dental compositions, with which the present invention is concerned, where far higher performance standards are required particularly as regards both cleaning of stained teeth and polishing.

Thus, prior art materials intended for use as cleaning and polishing constituents of prophylactic compositions have been unsatisfactory in one or more of the following respects, namely poor cleaning and polishing performances (especially with respect to inhibition of reaccumulation of dental calculus, pellicle, plaque, and exogenous stains), incompatibility with fluoride-containing anticariogenic agents, and adverse scratching and abrasion.

### Summary of invention

In accordance with the present invention, it has been found that new and more effective dental prophylactic preparations may be obtained by incorporating therein, as cleaning and polishing constituents, calcined particles of certain selected naturally occurring or synthetic magnesium silicate particles and specifically calcined particles of a synthetic or naturally occurring magnesium silicate or aluminosilicates selected from magnesium trisilicate, amorphous hydrous magnesium silicate, smectite, saponite, sepiolite or chlorite, the particles being predominantly in the magnesium metasilicate form. The particle size range is such that, substantially none of the calcined particles is retained on an 80 mesh screen (177 µm) and essentially all pass through a 100 mesh screen (149 µm). Desirably, all of the particles are less than about 150 µm in diameter, and advantageously the median particle size lies in the range of 10—40 µm.

It has been discovered that these selected silicate minerals, when calcined, preferably at a temperature in the range 800 to 1200°C, and when prepared in the desired particle size ranges, can be used as a combined cleaning and polishing agent, overcoming the aforementioned problems of the prior art. Tests

2

**0 048 264**

show that excellent cleaning and polishing results are obtained from a range of particle sizes within given limits, so that all particles participate in both functions, regardless of size. Cleaning and polishing are achieved with less scratching of the enamel surface than with prior materials. Also, it has been found that these larger particles of calcined magnesium metasilicate, in contrast to other dental prophylactic abrasives, are less abrasive than smaller particles of other materials, which accentuate the advantage.

It has further been found that the novel cleaning and polishing agents of the present invention may be used with non-toxic amounts of water-soluble anticariogenic adjuvants, such as sodium fluoride, stannous fluoride, sodium monofluorophosphate, or acidulated phosphate fluoride. In addition, with calcined magnesium silicates, the amount of anticariogenic agent added can be substantially reduced and yet superior reduction of enamel solubility is obtained. Such property has, among others, the advantage that taste problems are minimized or avoided.

It has likewise been discovered that application of prophylactic preparations of the present invention to the teeth provides a novel method for cleaning and polishing teeth and for reducing the incidence of gingivitis.

Through the use of the cleaning and polishing agents of the present invention, the difficulties experienced with prior art dental prophylactic cleaning and polishing agents may be overcome, and compositions of the present invention may therefore be used to formulate prophylactic pastes with superior cleaning and polishing capabilities and with enhanced anticariogenic ion compatibilities.

The naturally-occurring silicate minerals suitable for use in this invention are representatives of the phyllosilicate group of minerals. These are all structurally similar in that they are composed of superimposed layers of two-dimensional silica tetrahedra and two-dimensional octahedra consisting of two sheets of closely packed oxygens or hydroxyls in which aluminum, iron, or magnesium atoms are embedded in octahedral coordination. However, only the magnesium-rich members of the phyllosilicate group exhibit the unique properties of the subject invention, these being smectite (e.g. hectorite $(Mg_{3-x}Li_x)Si_4O_{10}(OH)_2$); saponite $(Mg_{3-x}Al_x)(Si_{4-y}Al_y)O_{10}(OH)_2)$); sepiolite (meerschaum $(Mg_2Si_3O_8 2H_2O)$; and chlorite $(Mg,Al,Fe)_6(Si,Al)_4O_{10}(OH)_8$.

As well as the naturally occurring minerals referred to above, the corresponding synthetic materials may be used, e.g. synthetic smectites similar in composition to saponite and hectorite, and including magnesium trisilicate $(Mg_2Si_3O_8)$, and amorphous hydrous magnesium silicate (empirical formula, $Mg_2Si_5O_{12}$). One preferred material is an amorphous hydrous magnesium silicate available under the trademark "Britesorb".

As used herein the term magnesium silicate should unless otherwise qualified be understood to refer to naturally occurring as well as synthetic magnesium silicates as described here.

While magnesium silicates containing some iron in their structure may be used for cleaning and polishing the teeth, the materials should be low in iron ions when combined with fluoride adjuvants for therapeutic treatment. Similarly, calcium-containing impurities such as dolomite and calcite, which occur frequently in deposits of talcs and clay minerals, must be removed from the magnesium silicate materials prior to calcination if a fluoride agent is to be added. Several naturally-occurring minerals which have been found to be particularly useful have approximate compositions by weight as in Table 1.

TABLE 1

Chemical compositions of naturally-occurring hydrous magnesium silicates

| Component | Per cent | | | |
| --- | --- | --- | --- | --- |
| | Chlorite | Hectorite | Saponite | Sepiolite |
| $SiO_2$ | 30.5 | 58.5 | 61.1 | 56.0 |
| $Al_2O_3$ | 22.4 | 0.2 | 9.3 | 4.0 |
| $Fe_2O_3$ | 2.1 | 0.2 | 0.9 | 1.0 |
| $MgO$ | 31.1 | 26.8 | 13.7 | 20.0 |
| $CaO$ | 0.15 | 1.1 | 2.7 | 0.5 |
| $Na_2O$ | 0.08 | 2.6 | 2.9 | 1.4 |
| $K_2O$ | 0.20 | 0.1 | 0.3 | 1.4 |
| $Li_2O$ | — | 1.2 | — | — |
| $TiO_2$ | 0.08 | — | 0.1 | — |

3

# 0 048 264

In their natural state all the described minerals are of little value as abrasive agents because of their softness, their colloid formation, swelling, gellation in water, and their reactivity, adsorpitivity, and ion exchangeability. However, thermal treatment of these minerals causes significant changes in their structures. At lower temperatures (usually 700°C. or lower) dehydration of most clay minerals is reversible because only adsorbed or interlayer water is lost and no structural changes occur. Heat treatment at higher temperatures (i.e. calcination) causes the minerals to undergo structural changes since hydroxyl water of the crystal lattice is lost and entirely new materials with totally different properties are produced. The types of alterations and the temperatures at which they occur vary with the chemical composition, structure and particle size of the mineral.

In accordance with this invention the magnesium silicates are calcined at a temperature lying in the range of about 800°C to 1200°C. Calcining may be achieved by heating in saggers in a furnace or by means of a rotary kiln wherein the degree of calcination may be controlled by altering the feed rate of material to the calciner, by varying the calcination residence time or the thickness of the material bed in the calciner, or by other methods known in the art. If the temperature does not reach 800°—900°C., the magnesium silicates remain in an incompletely dehydrated form, a material which is insufficiently hard to clean and polish satisfactorily from a dental standpoint. Material which has been calcined in the range of 800° to 1000°C. is predominantly magnesium metasilicate in the form of enstatite-type crystals. However, if more highly calcined (i.e. is subjected to temperatures of up to about 1200°C.), materials such as cristobalite ($SiO_2$) are formed. The amount of cristobalite formed is dependent on the ratio of magnesium to silicon in the starting material. The higher the magnesium content, the more favored is the production of the preferred metasilicate form. Magnesium metasilicate (enstatite) is the preferred reaction product because of its lower hardness and unique crystal structure. Material containing large amounts of cristobalite is unsatisfactory from a dental standpoint because of its tendency to abrade the tooth enamel unless reduced in size by milling or grinding. As a consequence, the calcined hydrous magnesium silicates of this invention are predominantly of the magnesium metasilicate (enstatite) form.

Smectites, because of the many possible substitutions and interlayer cations, show wide variations of temperature for the loss of hydroxyl lattice water. Some smectites which have a moderately high magnesium content show a lowering of the dehydration temperature. On the other hand, the magnesium-rich hectorite, in which the hydroxyls are partially replaced by fluorine, dehydrates at a higher temperature than the aluminous smectites.

As noted, the starting magnesium silicate minerals are ordinarily hydrous in nature. However, it is possible to employ dehydrated magnesium silicates in accordance with this invention since such materials are also transformed into the desired metasilicate form upon calcination regardless of the initial extent of hydration.

After calcining in the temperature range of 1000°C. to 1200°C., many of the materials agglomerate into large masses and grinding and/or milling are required to obtain an abrasive having a particle size distribution lying in the range found to be useful in dental prophylaxis compositions. Economical dry grinding processes such as conventional ball-milling may be employed, followed by screening through standard mesh sieves to separate incompletely degraded agglomerates. The preparation of suitably sized particles of the calcined magnesium silicates may also be accomplished by other conventional techniques well known to the art.

The calcined magnesium silicates of the present invention have a particle size range such that substantially none of the particles is retained on an 80 mesh screen (177 μm) and the size range is essentially less than 100 mesh (149 μm). Preferably, the particle size distribution falls into the ranges as shown in Table 2 which were determined by means of a Micromerograph.

4

# 0 048 264

TABLE 2
Particle size distribution range of calcined magnesium silicates

| Particle size (μm) | Weight (%) |
|---|---|
| >70 | 0—10 |
| 60—70 | 0—10 |
| 50—60 | 0—15 |
| 40—50 | 2—20 |
| 30—40 | 5—25 |
| 20—30 | 5—25 |
| 10—20 | 10—40 |
| 5—10 | 5—30 |
| <5 | 0—20 |
| Median | x=10—40 μm |

Cleaning and polishing without serious scratching are pronounced when the particle size range is such that a majority of the particles by weight pass through a 200 mesh (74 μm) screen. Within the indicated size range, the particles are effective for both cleaning and polishing. Hence, it is unnecessary to be meticulous in proportioning large and small particles so as to obtain a blend that can both clean and polish teeth. The value of this can be better understood by recognizing that for prophylactic cleaning and polishing of children's teeth, very little actual scouring is required, but polishing is important because immature teeth have a low luster. In contrast, whereas for adult's teeth, which are often stained by tobacco, coffee, tea, etc., substantial cleaning ability is required. With prior art cleaning and polishing agents, separate mixes of finer particle sizes are made to avoid excess and unnecessary scratching and abrasion of the teeth, and other mixes of larger sized particles are made to perform the difficult cleaning adequately.

With the calcined magnesium silicates of the present invention, it is possible to use a single overall size range of particles for both children's and adult's teeth. Moreover, in both situations the degree of abrasivity or scratching to the oral hard tissues is less than with prior art materials. Thus, this product is considerably safer to use than prior art materials, because it cleans and polishes as well or better than prior art materials with less deleterious abrasion and scratching.

The calcined magnesium silicate cleaning and polishing agents of the present invention may be applied directly to the teeth as a powder in aqueous slurry form. However, it is preferred that the agent be applied in the form of a prophylactic paste composition. The cleaning and polishing agent is provided in the paste composition within a range of about 30—95% by weight of the overall composition, depending upon the particular formulation desired, as is well known to one skilled in the art. Where desired, a portion of the calcined magnesium silicates of this invention or combinations thereof may be replaced by compatible fillers, extenders, or other abrasives such as uncalcined talcs, uncalcined magnesium silicates, aluminum silicates, alumina, zirconium silicate, insoluble sodium metaphosphate and silicas, and mixtures of these other agents as well as other dental abrasive materials. The prophylaxis paste may be prepared in a conventional manner and usually includes additional ingredients that render the overall composition commercially acceptable. For example, prophylaxis pastes typically contain conventional components such as water, binders, humectants, flavoring agents, sweeteners and detergents, in the range of up to approximately 50% by weight. Through the use of a prophylactic paste of the character described, it is possible to obtain clean, yet highly polished, oral hard tissues during the infrequent (i.e., semi-annual) professional prophylactic treatments performed by a dentist or dental hygienist.

Furthermore, it is preferred that anticariogenic agents be incorporated in such prophylactic pastes so that the advantages of such agents may be obtained in addition to the cleaning and polishing advantages of the abrasive component. The anticariogenic agent may comprise one or more water-soluble fluoride salts, including NaF, $SnF_2$, $Na_2PO_3F$ as well as acidulated phosphate fluoride (APF) mixtures. Other suitable fluoride adjuvants include KF, LiF, $SnF_4$, $InF_3$, $PbF_2$, $FeF_2$, $TiF_4$, and $NH_4F$, as well as more complex water-soluble fluoride-containing adjuvants such as fluorosilicates, e.g. $Na_2SiF_6$; fluorozirconates, e.g. $CaZrF_6$, $Na_2ZrF_6$, $K_2ZrF_6$, $SnZrF_6$, $InZrF_7$; fluorostanites, e.g. $NaSnF_3$, $KSnF_3$, $NaSn_2F_5$; fluoroborates, e.g. $NaBF_4$; fluorotitanates, e.g. $NaTiF_5$; fluorogermanates, e.g. $K_2GeF_6$, $Zr(GeF_6)_2$, $ZrOGeF_6$, $In_2(GeF_6)_3$; and mixed halides, e.g. SnClF and $Sn_2ClF_3$.

5

# 0 048 264

When used in combination with a fluoride-containing anticariogenic adjuvant in aqueous solution, the tendency of the calcined magnesium silicates of the present invention to react with or deactivate the fluoride adjuvant is substantially nil. Because of the compatibility of these cleaning and polishing agents with fluoride compounds throughout the biologically feasible pH range, the anticariogenic effect of the fluoride can be obtained using lower concentrations of the adjuvant than heretofore possible with prior art dental prophylactic abrasives. This eliminates or at least reduces bad flavor problems and the nauseating effects of conventional larger quantities of fluoride-containing adjuvants, especially stannous fluorides.

Fluoride-containing adjuvants are employed in pastes of the invention at a non-toxic concentration sufficient to significantly reduce the incidence of dental caries in patients. This concentration may range widely, and depends, at least in part, upon the nature of the chosen adjuvant. In general, satisfactory results may be obtained within the range of about 0.1 to 20% by weight of the paste composition (calculated as fluoride ion). When NaF, $Na_2PO_3F$, or HF are utilized, such compounds are preferably employed at levels of about 0.5 to 5% by weight of the paste. With $SnF_2$ a concentration range of approximately 1 to 10% is acceptable.

Advantageously, a source of stable phosphate (e.g. phosphoric acid or sodium orthophosphates) may be used in conjunction with the fluoride agent in order to enhance its anticariogenic activity.

In addition, other suitable anticariogenic adjuvants, such as the aluminum carboxylate complexes described in U.S. Patents Nos. 4,042,680 and 4,108,981 and other non-toxic water soluble sources of aluminum ions may also be employed to produce anticariogenic prophylactic paste compositions in accordance with the subject invention.

Compositions of exemplary prophylactic paste preparations employing the cleaning and polishing agents of the present invention are given in the following examples:

Example 1

| Constituent | % by weight |
|---|---|
| Sepiolite (Calcined at 1100°C.) | 45.0 |
| Distilled Water | 20.9 |
| Glycerin | 15.0 |
| Sorbitol (70% aqueous solution) | 16.0 |
| Veegum (Magnesium aluminum silicate) | 0.5 |
| Sodium Carboxymethyl Cellulose | 1.0 |
| Sodium Saccharin | 0.5 |
| Flavor | 1.0 |
| Methyl p-hydroxybenzoate | 0.1 |
| | 100.0% |

The above composition may be mixed to any consistency desired with water or fluoride solution immediately before use according to the preferences of the dentist or dental hygienist.

6

### Example 2

| Constituent | % by weight |
|---|---|
| Synthetic Amorphous Hydrous Magnesium Silicate (calcined at 1100°C) | 44.0 |
| Distilled Water | 24.4 |
| Propylene Glycol | 13.0 |
| Sorbitol (70% aqueous solution) | 13.0 |
| Laponite (Synthetic colloidal magnesium silicate) | 0.8 |
| Sodium Carboxymethyl Cellulose | 0.7 |
| Sodium Saccharin | 0.5 |
| Flavor | 1.0 |
| Sodium Lauryl Sulfate | 0.5 |
| Methyl p-hydroxybenzoate | 0.1 |
| Sodium Fluoride, NaF | 2.0 |
| | 100.0% |

### Example 3

| Constituent | % by weight |
|---|---|
| Hectorite (calcined at 900°C.) | 52.1 |
| Distilled Water | 18.0 |
| Propylene Glycol | 20.0 |
| Hydroxyethyl Cellulose | 0.9 |
| Sodium Saccharin | 1.0 |
| Flavor | 2.0 |
| Trisodium Citrate | 1.0 |
| Stannous Fluoride, $SnF_2$ | 5.0 |
| | 100.0% |

# 0 048 264

Example 4

| Constituent | % by weight |
|---|---|
| Magnesium Trisilicate (calcined at 1000°C.) | 51.0 |
| Distilled Water | 19.5 |
| Glycerin | 4.7 |
| Propylene Glycol | 12.5 |
| Sorbitol (70% aqueous solution) | 2.7 |
| Bentone (Organo-clay gellant) | 1.5 |
| Sodium Saccharin | 0.4 |
| Flavor | 0.6 |
| Sodium Fluoride, NaF | 4.0 |
| Sodium Dihydrogen Phosphate, $NaH_2PO_4$ | 2.3 |
| Miscellaneous | 0.8 |
| | 100.0% |

Experimental evaluations

The superiority of the calcined magnesium silicate prophylactic cleaning and polishing agents disclosed herein as compared with other abrasives has been substantiated by the following experimental evaluations.

Testing was performed with an instrument designed specifically for the purpose of evaluating prophylactic compositions. This device has an adjustable velocity motor-mandrel assembly to which the prophylactic cup is attached, the entirety of which can be automatically moved laterally back and forth by means of a flexible shaft-step motor arrangement. The extent of the lateral movement is precisely controlled by limit switches adjustable to 0.01 inch (254 μm). The specimen is positioned in a round cup and is held in place by means of a permanent magnet. The specimen cup is revolved by means of a flexible shaft-variable speed motor assembly, and is positioned on the pan end of a triple-beam balance that .enables the precise adjustment of pressure. The prophylactic cup motor assembly is lowered onto the specimen and the height of the specimen cup is adjusted by means of a screw until the force is sufficient to balance the weighted arm of the triple-beam balance. During treatment the prophylactic cup is automatically raised every ten seconds from the specimen surface to simulate slurry replenishment during prophylaxis. For the polishing and abrasion evaluations abrasive slurries were prepared by mixing two parts of a 1% sodium carboxymethyl cellulose solution with one part of abrasive by weight in order to prevent the abrasive from settling.

Polishing evaluations were made with bovine permanent incisors mounted in Wood's metal with the labial surface exposed. The labial surface was leveled by means of a mechanical surface grinder so as to provide a smooth, uniform area for testing that was not into the dentin and was parallel to the base. The teeth selected were of sufficient size to provide a leveled area approximately 1.0 cm in diameter. The mounted tooth specimens were dulled by immersion in 0.2M HCl for thirty seconds, followed by thorough rinsing with distilled water. The specimens were mechanically treated by means of a simulated prophylaxis where the prophylactic cup was rotated under a load of 300 grams of 1000 rpm and oscillated ±0.14 inch (3.6 mm) from the zero position at a rate of five cycles per minute, and the specimen cup revolved at 30 rpm. This procedure produced a uniformly randomized treatment pattern that ensured reproducibility between replicates. Treatment times were varied from ten seconds to cumulative times of twelve minutes.

The reflectance of the polished tooth surface was determined by means of a reflectometer designed to detect changes in the degree of luster of the enamel surface. This instrument produces a beam of light which, when reflected from the leveled tooth surface, impinges on a photoelectric cell which in turn activates an X—Y recorder, producing a graphical print-out of the entire leveled tooth surface. The smoother and more highly polished the enamel surface, the smaller is the amount of diffused and absorbed light and, hence, the higher the reflectance reading. The reflectometer was calibrated so that 0 represented total darkness and 100 was set to white carrara glass standards, and the data for the abrasives tested hereinafter are reported on this scale.

8

**0 048 264**

The harmfulness of a prophylactic abrasive can be expressed in terms of dentin and enamel abrasion values. Dentin and enamel abrasion values for prophylaxis paste cleaning and polishing agents were determined using bovine teeth. Dentin specimens were prepared by sagittally sectioning bovine permanent incisors through the pulp cavity, carefully trimming off the enamel around the perimeter, and mounting the anterior half in a block of self-curing acrylic with the exposed dentin facing upward. The dentin was ground flush with the acrylic surface using a model trimmer and a coarse wheel. The ground surface was then uniformly smoothed and leveled with a mechanical surface grinder. The dentin specimens were treated for a total of two minutes using the prophylaxis instrument as described previously for enamel polish. Enamel abrasion specimens were prepared by immersing trimmed bovine incisors in dental acrylic with the labial surface facing upward. The blocks were ground, using a model trimmer and coarse grinding wheel, until the enamel was leveled to an area approximately 9 mm in length and was flush with the acrylic. Further smoothing and flattening were accomplished with a mechanical surface grinder, then a very smooth, optically polished surface was produced using 0.3 micron alpha alumina on a horizontal polishing wheel with a silk cover. The enamel specimens were treated for a total of five minutes using the prophylaxis instrument to administer a reciprocating treatment wherein the specimen was not revolved but maintained stationary while the prophylactic cup made repeated passes over it. All other treatment conditions were the same as described previously.

The amount of dentin or enamel removed by each abrasive under identical treatment conditions was used as an indicator of abrasivity and was quantitatively measured by means of a proficorder, a surface profile measuring device. The proficorder has a diamond stylus which, when tracing the surface of a specimen, produces an electrical signal proportional to the irregularities on the traced surface. These signals are amplified and converted into a graphical form on a strip chart producing a very sensitive, accurate record of the surface microtopography. By superimposing before-and-after treatment traces, the cross-sectional area of abrasion was visualized and was measured by means of a planimeter. From this value the mean abraded depth of each specimen was calculated, and both the dentin and enamel abrasion data are hereinafter reported in this fashion.

The effectiveness of a dental abrasive as a compatible carrier vehicle for fluoride-containing adjuvants was determined by measuring the amounts of available fluoride and tin(II) ions in solution. Percentage availability refers to a comparison of an ionic concentration level with a reference solution of the adjuvant without the carrier vehicle. A percentage ratio of the ionic concentration level detected for each abrasive agent was determined by adding 8.00 grams of abrasive to a 20 ml aliquot of 1000 ppm fluoride solution, mechanically shaking for fifteen minutes, centrifuging until clear, and decanting. The supernatant was analyzed for fluoride with a fluoride electrode and for tin(II) by means of an iodimetric titration. Thus, for example, a combination solution of abrasive (carrier vehicle) and sodium fluoride which analyzed 900 ppm fluoride concentration compared to a reference solution of sodium fluoride at 1000 ppm fluoride exhibits at 90% availability.

Enamel polish data were obtained for a number of prophylactic abrasives in accordance with this invention. For comparative purposes, data were also obtained for several conventional prophylactic abrasives as well as a number of magnesium compounds not encompassed by this invention. These data, which are provided in Table 3, show the superior polishing characteristics of the calcined magnesium silicates of this invention.

9

# 0 048 264

## TABLE 3
### Enamel polish of calcined magnesium silicates and other abrasives

| Polishing agent | Calcining temperature | Polish score (30-second treatment) mean±standard error |
|---|---|---|
| Amorphous Hydrous Magnesium Silicate | Uncalcined 900°C | 64±2 87±3 |
| Hectorite | 900°C | 99±3 |
| Magnesium Trisilicate | Uncalcined 900°C | 48±6 89±5 |
| Sepiolite | Uncalcined 1000°C | 35±0 103±3 |
| Chlorite | Uncalcined 1200°C | 38±2 91±3 |
| Saponite | 900° | 94±2 |
| Cristobalite, $SiO_2$ | — | 62±5 |
| Fieldspar, $(Na,K)AlSi_3O_8$ | — | 76±3 |
| Magnesium Carbonate, $MgCO_3$ | — | 45±3 |
| Magnesium Hydroxide, $Mg(OH)_2$ | — | 40±3 |
| Magnesium Oxide, MgO | — | 62±2 |
| Magnesium Phosphate, $Mg_3(PO_4)_2$ | — | 49±1 |
| Pumice, Coarse | — | 53±1 |
| Pumice, Fine | — | 67±3 |
| Pumice, Flour | — | 72±2 |
| Quartz, $SiO_2$ | — | 65±3 |
| Zirconium Silicate, $ZrSiO_4$ | — | 74±2 |

Several of these polishing agents and a number of conventional prophylactic abrasives were also examined for their rate of enamel polishing ability (i.e. polish versus treatment time). These data, which are reported in Table 4, demonstrate that the materials of this invention produce more rapid rates of polish and higher luster maximums than conventional abrasives used for dental prophylaxis.

10

TABLE 4
Enamel polish rate of calcined magnesium silicates and other abrasives

| Polishing agent | Calcining temperature | Polish score (vs. treatment time in minutes) | | | | | | |
|---|---|---|---|---|---|---|---|---|
| | | 1/6 | 1/2 | 1 | 2 | 4 | 8 | 12 |
| Amorphous Hydrous Magnesium Silicate | 1000°C. | 52 | 98 | 106 | 109 | 112 | 112 | 114 |
| Hectorite | 900°C. | 81 | 100 | 107 | 113 | 114 | 119 | 120 |
| Magnesium Trisilicate | 900°C. | 51 | 90 | 100 | 106 | 111 | 113 | 115 |
| Sepiolite | 1000°C. | 80 | 103 | 112 | 116 | 116 | 119 | 118 |
| Cristobalite | — | 39 | 70 | 81 | 90 | 98 | 102 | 105 |
| Feldspar | — | 50 | 76 | 89 | 98 | 105 | 108 | 109 |
| Pumice, Coarse | — | 32 | 54 | 69 | 84 | 87 | 94 | 94 |
| Pumice, Fine | — | 46 | 64 | 79 | 89 | 96 | 97 | 100 |
| Pumice, Flour | — | 51 | 75 | 86 | 92 | 98 | 103 | 105 |
| Quartz | — | 45 | 71 | 90 | 102 | 106 | 107 | 111 |
| Zirconium Silicate | — | 64 | 85 | 98 | 106 | 111 | 113 | 115 |

Enamel and dentin abrasion data were obtained for several calcined magnesium silicates and conventional prophylaxis abrasives and are reported in Table 5. The data in Table 5 show that the materials of this invention are of low abrasivity to both enamel and dentin.

TABLE 5
Enamel and dentin abrasion of calcined magnesium silicates and other abrasives

| Polishing agent | Calcining temperature | Enamel abrasion score (micro-in.) mean± standard error | Dentin abrasion score (micro-in.) mean± standard error |
|---|---|---|---|
| Amorphous Hydrous Magnesium Silicate | 1000°C. | 81±16 | 2031±283 |
| Sepiolite | 1000°C. | 38±11 | 715±81 |
| Feldspar | — | 90±40 | 1832±151 |
| Pumice, Coarse | — | 967±114 | 3245±205 |
| Pumice, Fine | — | 822±67 | 3068±478 |
| Pumice, Fluor | — | 476±161 | 2634±327 |
| Zirconium Silicate | — | 269±57 | 1861±55 |

Fluoride availability data were determined for a number of prophylaxis abrasives in accordance with this invention. For comparison, data were also obtained for conventional prophylaxis abrasives and several magnesium compounds not included in this invention. These data, which are provided in Table 6, demonstrate the high degree of compatibility of the calcined magnesium silicates with three different fluoride-containing adjuvants.

TABLE 6
Fluoride compatibility of calcined magnesium silicates and other abrasives

| Polishing agent | Calcining temperature | Per cent availability | | | |
|---|---|---|---|---|---|
| | | F (as NaF) | F (as APF) | F (as SnF$_2$) | Sn++ (as SnF$_2$) |
| Amorphous Hydrous Magnesium Silicate | Uncalcined | 81.3 | 34.5 | 67.2 | 0.2 |
| | 1000°C | 99.7 | 100.3 | 97.7 | 96.7 |
| Hectorite | 1100°C | 100.4 | 94.8 | 99.2 | 91.8 |
| Magnesium Trisilicate | Uncalcined | 25.1 | 5.8 | 12.4 | 0.2 |
| | 1100°C | 99.3 | 99.6 | 98.7 | 83.8 |
| Sepiolite | Uncalcined | 43.8 | 31.3 | 31.6 | 0.2 |
| | 1100°C | 99.9 | 84.1 | 96.9 | 90.0 |
| Chlorite | Uncalcined | 73.0 | 48.6 | 65.3 | 36.4 |
| | 1200°C | 100.2 | 52.4 | 90.4 | 77.6 |
| Saponite | 1000°C | 99.7 | 80.6 | 98.8 | 92.6 |
| Christobalite | — | 98.3 | N.D. | 97.2 | N.D. |
| Feldspar | — | 99.3 | 21.3 | 88.8 | 59.1 |
| Magnesium Carbonate | — | 49.2 | 12.1 | 8.5 | 0.2 |
| Magnesium Hydroxide | — | 7.6 | 25.2 | 1.7 | 0.2 |
| Magnesium Oxide | — | 0.5 | 9.0 | 1.0 | 3.9 |
| Magnesium Phosphate | — | 69.3 | 17.6 | 31.5 | 0.6 |
| Pumice, Coarse | — | 98.8 | 86.7 | 96.6 | 71.8 |
| Pumice, Fine | — | 97.8 | 85.9 | 96.1 | 70.9 |
| Pumice, Flour | — | 89.3 | 68.5 | 81.3 | 56.7 |
| Quartz | — | 96.2 | N.D. | 96.3 | N.D. |
| Zirconium Silicate | — | 99.1 | N.D. | 93.9 | N.D. |

N.D.—Not Determined

The foregoing data are supportive of the significant improvement in dental health that may be achieved by utilizing dental prophylactic compositions containing the calcined magnesium silicate cleaning and polishing agents of this invention.

**Claims**

1. A dental prophylactic composition comprising as its principal cleansing and polishing component calcined particles of an inorganic silicate or aluminosilicate having a particle size below 80 mesh, U.S. Sieve Series (0.18 mm) and a median particle size in the range 10—40 µm, characterised in that said particles are calcined particles of a synthetic or naturally occurring phyllosilicate mineral selected from magnesium trisilicate, amorphous hydrous magnesium silicate, smectite, sepiolite, saponite or chlorite, said particles

12

being predominently in the magnesium metasilicate form, and obtained by calcination of the phyllosilicate mineral at a temperature in the range 800—1200°C.

2. A dental prophylactic composition according to claim 1 additionally containing a water-soluble, non-toxic anticariogenic adjuvant selected from water-soluble fluoride containing salts and water-soluble aluminium ion-containing salts.

3. A dental prophylactic composition according to claim 1 or 2, characterised in that said calcined particles are present in an amount of from 30—95% by weight based on the total weight of the composition.

**Revendications**

1. Une composition de prophylaxie dentaire comprenant comme composant principal de nettoyage et de polissage, des particules calcinées d'un silicate ou aluminosilicate minéral ayant une taille des particules inférieure à 80 mesh, selon l'U.S. Sieve Series (0,18 mm) et une taille médiane des particules dans la gamme de 10—40 µm, caractérisée en ce que lesdites particules sont des particules calcinées d'un minéral phyllosilicate synthétique ou naturel, choisi parmi le trisilicate de magnésium, le silicate de magnésium hydraté amorphe, la smectite, la sépiolite, la saponite ou la chlorite, lesdites particules étant de façon prédominante sous forme de métasilicate de magnésium et obtenues par calcination du minéral à structure de phyllosilicate à une température dans la gamme de 800 à 1200°C.

2. Une composition de prophylaxie dentaire selon la revendication 1, contenant de plus un adjuvant anticariogène non toxique, soluble dans l'eau, choisi parmi les sels solubles dans l'eau contenant du fluorure et les sels solubles dans l'eau contenant l'ion aluminium.

3. Une composition de prophylaxie dentaire selon la revendication 1 ou 2, caractérisée en ce que lesdites particules calcinées sont présentes en une quantité de 30 à 95% en poids par rapport au poids total de la composition.

**Patentansprüche**

1. Zahnprophylaktische Zusammensetzung, enthaltend als Hauptreinigungs- und-polierkomponente calcinierte Teilchen eines anorganischen Silicats oder Aluminosilicats mit einer Teilchengrösse unter 80 mesch, U.S. Sieve Series (0,18 mm) und einer mittleren Teilchengrösse im Bereich von 10—40 µm, dadurch gekennzeichnet, dass diese Teilchen calcinierte Teilchen eines synthetischen oder natürlich vorkommenden Phyllosilicatminerals, ausgewählt aus Magnesiumtrisilicat, amorphem wässerigem Magnesiumsilicat, Smectit, Sepiolit, Saponit oder Chlorit, sind und die Teilchen vorwiegend in der Magnesium-metasilicatform vorliegen und erhalten werden durch Calcinieren des Phyllosilicatminerals bei einer Temperatur im Bereich von 800—1200°C.

2. Zahnprophylaktische Zusammensetzung nach Anspruch 1, die zusätzlich ein wasserlösliches, nicht-toxisches antikarzinogenes Adjuvans enthält, das augewählt ist aus wasserlöslichen, Fluorid enthaltenden Salzen und wasserlöslichen, Aluminiumionen enthaltenden Salzen.

3. Zahnprophylaktische Zusammensetzung nach Anspruch 1 oder 2, dadurch gekennzeichnet, dass die calcinierten Teilchen in einer Menge von 30—95 Gew.-%, basierend auf dem Gesamtgewicht der Zusammensetzung, vorhanden sind.